(19) Europäisches Patentamt
European Patent Office
Office européen des brevets

(11) Veröffentlichungsnummer: **0 661 276 A1**

(12) # EUROPÄISCHE PATENTANMELDUNG

(21) Anmeldenummer: **94116723.1**

(22) Anmeldetag: **24.10.94**

(51) Int. Cl.6: **C07D 239/545**, A01N 47/36

(30) Priorität: **05.11.93 DE 4337847**

(43) Veröffentlichungstag der Anmeldung:
**05.07.95 Patentblatt 95/27**

(84) Benannte Vertragsstaaten:
**BE CH DE ES FR GB IT LI NL**

(71) Anmelder: **BAYER AG**

**D-51368 Leverkusen (DE)**

(72) Erfinder: **Riebel, Hans-Jochem, Dr.**
**In der Beek 92**
**D-42113 Wuppertal (DE)**
Erfinder: **Drewes, Mark Wilhelm, Dr.**
**Goethesrasse 38**
**D-40764 Langenfeld (DE)**
Erfinder: **Jansen, Johannes R., Dr.**
**Knipprather Strasse 47**
**D-40789 Monheim (DE)**
Erfinder: **Dollinger, Markus, Dr.**
**Burscheider Strasse 154b**
**D-51381 Leverkusen (DE)**
Erfinder: **Santel, Hans-Joachim, Dr.**
**Grünstrasse 9a**
**D-51371 Leverkusen (DE)**

(54) **Substituierte Phenylaminosulfonylharnstoffe mit herbizider Wirkung.**

(57) Die Erfindung betrifft neue substituierte Phenylaminosulfonylharnstoffe der Formel (I),

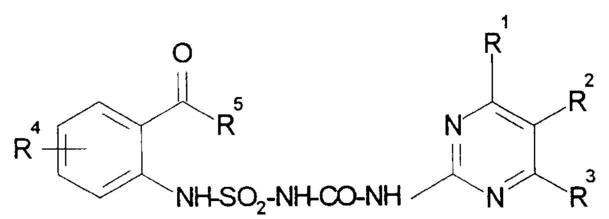

(I)

in welcher $R^1$ bis $R^4$ die in der Beschreibung angegebenen Bedeutungen haben und

$R^5$ für gegebenenfalls durch Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 8 Kohlenstoffatomen, für jeweils gegebenenfalls durch Halogen und/oder $C_1$-$C_4$-Alkyl substituiertes Cycloalkyl, Cycloalkyl-$C_1$-$C_4$-alkyl, Cycloalkenyl oder Cycloalkenyl-$C_1$-$C_4$-alkyl mit jeweils 4 bis 7 Kohlenstoffatomen in den Cycloalkyl- oder Cycloalkenylgruppen, für jeweils gegebenenfalls durch Halogen, $C_1$-$C_4$-Alkyl oder $C_1$-$C_4$-Halogenalkyl substituiertes Phenyl oder Phenyl-$C_1$-$C_4$-alkyl oder für jeweils gegebenenfalls durch Halogen, $C_1$-$C_4$-Alkyl oder $C_1$-$C_4$-Halogenalkyl substituiertes Heterocyclyl oder Heterocyclyl-$C_1$-$C_4$-alkyl mit 2 bis 6 Kohlenstoffatomen und 1 bis 3 Stickstoffatomen und/oder einem Sauerstoff- oder Schwefelatom steht,

sowie Salze der Verbindungen der Formel (I), Verfahren zur Herstellung dieser Verbindungen und deren Verwendung als Herbizide.

EP 0 661 276 A1

Die Erfindung betrifft neue substituierte Phenylaminosulfonylharnstoffe, Verfahren Zu ihrer Herstellung und ihre Verwendung als Herbizide.

Es ist bekannt, daß die Verbindung N-(4,6-Dimethoxy-pyrimidin-2-yl)-N'-(2-cyclo-propy-carbonyl-phenylaminosulfonyl)-harnstoff, als selektives Herbizid eingesetzt werden kann (vgl. EP-A 463287 / US-P 5009699).

Einige Alkylcarbonylphenylaminosulfonylharnstoffe sind aus älteren Patentanmeldungen bekannt, haben jedoch keine Bedeutung erlangt (vgl. EP-A 264467 / US-P 4622065).

Es wurden nun die neuen substituierten Phenylaminosulfonylharnstoffe der allgemeinen Formel (I)

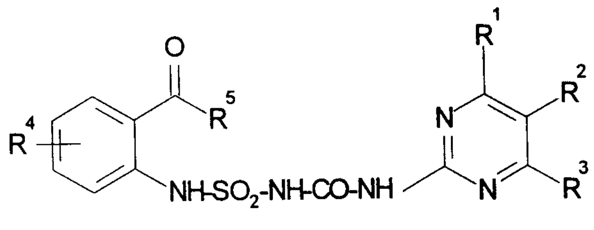

(I)

in welcher

$R^1$ für Wasserstoff, Halogen oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylamino oder Dialkylamino mit jeweils 1 bis 4 Kohlenstoffatomen in den einzelnen Alkylgruppen steht,

$R^2$ für Wasserstoff oder Halogen steht,

$R^3$ für Halogen oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylamino oder Dialkylamino mit jeweils 1 bis 4 Kohlenstoffatomen in den einzelnen Alkylgruppen steht,

$R^4$ für Wasserstoff, Amino, Halogen, Formyl oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylcarbonyl, Alkoxycarbonyl, Alkylcarbonylamino, Alkoxycarbonylamino oder Alkylsulfonylamino mit jeweils 1 bis 4 Kohlenstoffatomen in den einzelnen Alkylgruppen steht, und

$R^5$ für Wasserstoff oder für gegebenenfalls durch Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 8 Kohlenstoffatomen, für jeweils gegebenenfalls durch Halogen und/oder $C_1$-$C_4$-Alkyl substituiertes Cycloalkyl, Cycloalkyl-$C_1$-$C_4$-alkyl, Cycloalkenyl oder Cycloalkenyl-$C_1$-$C_4$-alkyl mit jeweils 4 bis 7 Kohlenstoffatomen in den Cycloalkyl- oder Cycloalkenylgruppen, für jeweils gegebenenfalls durch Halogen, $C_1$-$C_4$-Alkyl oder $C_1$-$C_4$-Halogenalkyl substituiertes Phenyl oder Phenyl-$C_1$-$C_4$-alkyl oder für jeweils gegebenenfalls durch Halogen, $C_1$-$C_4$-Alkyl oder $C_1$-$C_4$-Halogenalkyl substituiertes Heterocyclyl oder Heterocyclyl-$C_1$-$C_4$-alkyl mit 2 bis 6 Kohlenstoffatomen und 1 bis 3 Stickstoffatomen und/oder einem Sauerstoff- oder Schwefelatom steht,

sowie Salze der Verbindungen der Formel (I) gefunden.

Man erhält die neuen substituierten Phenylaminosulfonylharnstoffe der allgemeinen Formel (I), wenn man Aminopyrimidine der allgemeinen Formel (II),

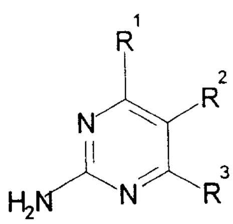

(II)

in welcher

$R^1$, $R^2$ und $R^3$ die oben angegebene Bedeutung haben,

mit Chlorsulfonylisocyanat, gegebenenfalls in Gegenwart eines Verdünnungsmittels, umsetzt und die hierbei gebildeten Chlorsulfonylharnstoffe der allgemeinen Formel (III),

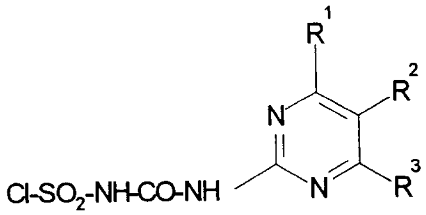

(III)

in welcher

$R^1$, $R^2$ und $R^3$ die oben angegebene Bedeutung haben,

mit Arylaminen der allgemeinen Formel (IV),

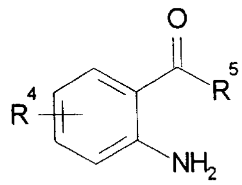

(IV)

in welcher

$R^4$ und $R^5$ die oben angegebene Bedeutung haben,

gegebenenfalls in Gegenwart eines Säureakzeptors und gegebenenfalls in Gegenwart eines Verdünnungsmittels umsetzt und gegebenenfalls die so erhaltenen Verbindungen der Formel (I) nach üblichen Methoden in Salze überführt.

Eine weitere mögliche Herstellungsmethode tur die erfindungsgemäßen Verbindungen der Formel (I) ist nachstehend skizziert, wobei $R^1$, $R^2$, $R^3$, $R^4$ und $R^5$ die oben angegebene Bedeutung haben und R für Alkyl (insbesondere für Methyl oder Ethyl), Aralkyl (insbesondere Benzyl) oder Aryl (insbesondere Phenyl) steht:

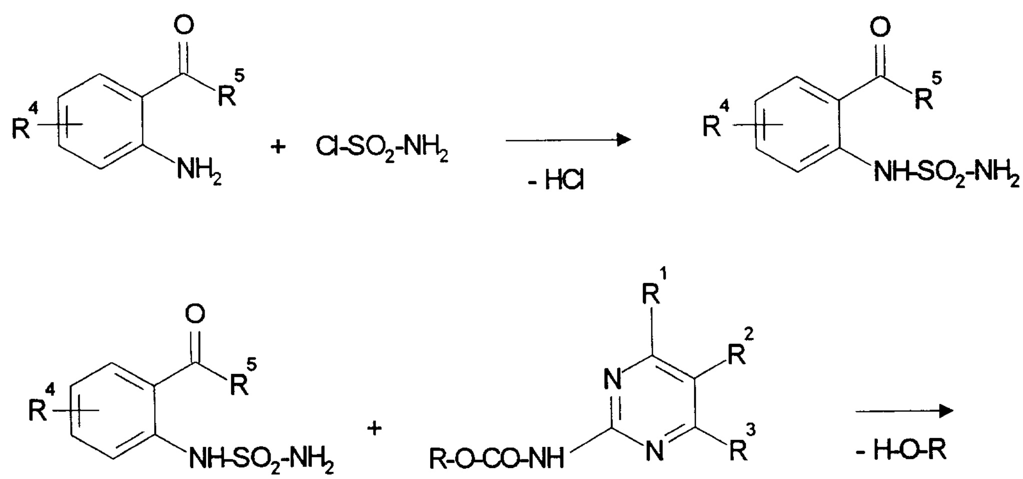


(I)

Die neuen substituierten Phenylaminosulfonylharnstoffe der allgemeinen Formel (I) zeichnen sich durch starke und selektive herbizide Wirksamkeit aus.

Überraschenderweise zeigen die neuen Verbindungen der Formel (I) erheblich stärkere herbizide Wirkung als die strukturell ähnliche bekannte Verbindung N-(4,6-Dimethoxy-pyrimidin-2-yl)-N'-(2-cyclopropyl-carbonyl-phenylaminosulfonyl)-harnstoff.

Gegenstand der Erfindung sind vorzugsweise Verbindungen der Formel (I), in welcher

$R^1$ für Wasserstoff, Fluor, Chlor, Brom oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylamino oder Dialkylamino mit jeweils 1 bis 3 Kohlenstoffatomen in den einzelnen Alkylgruppen steht,

$R^2$ für Wasserstoff Fluor, Chlor oder Brom steht,

$R^3$ für Fluor, Chlor, Brom oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylamino oder Dialkylamino mit jeweils 1 bis 3 Kohlenstoffatomen in den einzelnen Alkylgruppen steht,

$R^4$ für Wasserstoff Amino, Fluor, Chlor, Brom, Formyl oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylcarbonyl, Alkoxycarbonyl, Alkylcarbonylamino, Alkoxycarbonylamino oder Alkylsulfonylamino mit jeweils 1 bis 3 Kohlenstoffatomen in den einzelnen Alkylgruppen steht, und

$R^5$ für Wasserstoff oder für gegebenenfalls durch Fluor, Chlor, Brom, $C_1$-$C_3$-Alkoxy, $C_1$-$C_3$-Alkylthio, $C_1$-$C_3$-Alkylsulfinyl oder $C_1$-$C_3$-Alkylsulfonyl substituiertes Alkyl mit 1 bis 6 Kohlenstoffatomen, für gegebenenfalls durch Fluor, Chlor, Brom und/oder $C_1$-$C_3$-Alkyl substituiertes Cycloalkyl, Cycloalkyl-$C_1$-$C_3$-alkyl, Cycloalkenyl oder Cycloalkeny-$C_1$-$C_3$-alkyl mit jeweils 4 bis 6 Kohlenstoffatomen in den Cycloalkyl- oder Cycloalkenylgruppen, für jeweils gegebenenfalls durch Fluor, Chlor, Brom, $C_1$-$C_3$-Alkyl oder $C_1$-$C_3$-Halogenalkyl substituiertes Phenyl oder Phenyl-$C_1$-$C_3$-alkyl oder für jeweils gegebenenfalls durch Fluor, Chlor, Brom, $C_1$-$C_3$-Alkyl oder $C_1$-$C_3$-Halogenalkyl substituiertes Heterocyclyl oder Heterocyclyl-$C_1$-$C_3$-alkyl mit 3 bis 5 Kohlenstoffatomen und 1 bis 3 Stickstoffatomen und/oder einem Sauerstoff- oder Schwefelatom steht.

Gegenstand der Erfindung sind weiter vorzugsweise Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, $C_5$- oder $C_6$-Cycloalkyl-ammonium- und Di-($C_1$-$C_2$-alkyl)-benzyl-ammonium-Salze von Verbindungen der Formel (I), in welcher $R^1$, $R^2$, $R^3$, $R^4$, und $R^5$ die oben vorzugsweise angegebenen Bedeutungen haben.

Gegenstand der Erfindung sind insbesondere Verbindungen der Formel (I), in welcher

$R^1$ für Wasserstoff Chlor, Methyl, Ethyl, Trifluormethyl, Methoxy, Ethoxy, Difluormethoxy, Methylthio, Ethylthio, Methylamino, Ethylamino oder Dimethylamino steht,

$R^2$ für Wasserstoff steht,

$R^3$ für Chlor, Methyl, Ethyl, Trifluormethyl, Methoxy, Ethoxy, Difluormethoxy, Methylthio, Ethylthio, Methylamino, Ethylamino oder Dimethylamino steht,

$R^4$ für Wasserstoff Amino, Fluor, Chlor, Brom, Methyl, Trifluormethyl, Methoxy, Methylthio, Methylsulfinyl, Methylsulfonyl, Acetyl oder Methoxycarbonyl steht, und

$R^5$ für Wasserstoff oder für jeweils gegebenenfalls durch Fluor und/oder Chlor substituiertes Methyl, Ethyl, n- oder i-Propyl, n-, i- oder s-Butyl, n-, i- oder s-Pentyl, für Methoxymethyl, Methoxyethyl, Ethoxymethyl, Ethoxyethyl, Methylthiomethyl, Methylthioethyl, Ethylthiomethyl, Ethylthioethyl, Methylsulfinylmethyl, Ethylsulfinylmethyl, Methylsulfonylmethyl, Ethylsulfonylmethyl, oder für jeweils gegebenenfalls durch Fluor, Chlor und/oder Methyl substituiertes Cyclobutyl, Cyclobutylmethyl, Cyclopentyl, Cyclopentylmethyl, Cyclopentenylmethyl, Cyclohexyl, Cyclohexenylmethyl, Phenyl, Benzyl, Phenylethyl, Furyl, Furylmethyl, Thienyl, Thienylmethyl, Tetrahydrofuryl oder Tetrahydrofurylmethyl steht.

Eine besonders bevorzugte Gruppe erfindungsgemäßer Verbindungen sind die Verbindungen der nachstehenden allgemeinen Formel (Ia), in welcher die Reste $R^1$, $R^2$, $R^3$, $R^4$ und $R^5$ die oben als insbesondere bevorzugt angegebene Bedeutung haben.

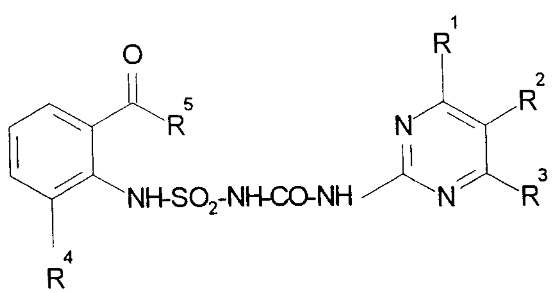

(Ia)

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man beispielsweise 4-Chlor-6-methoxy-2-amino-pyrimidin, Chlorsulfonylisocyanat und 2-Propionyl-anilin als Ausgangsstoffe, so kann der Reaktionsablauf beim erfindungsgemäßen Verfahren durch das folgende Formelschema skizziert werden:

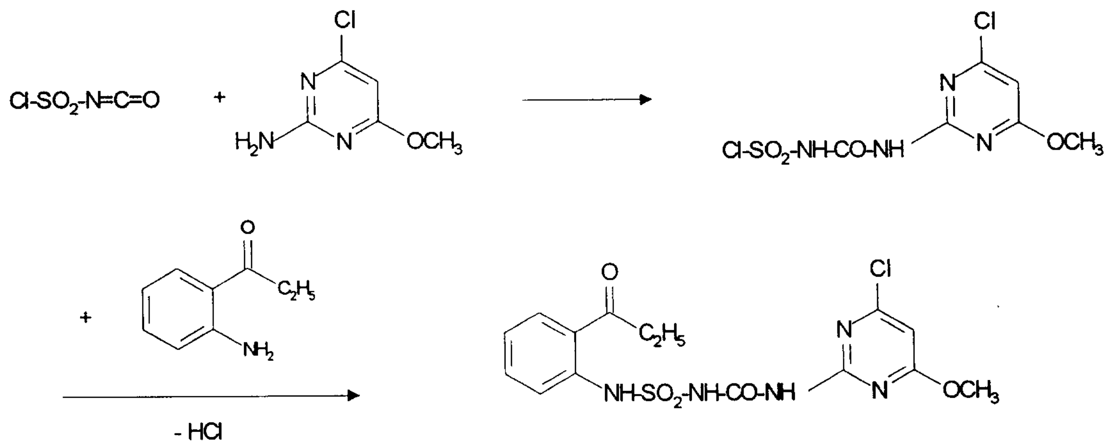


Die beim erfindungsgemäßen Verfahren als Ausgangsstoffe zu verwendenden Aminopyrimidine der Formel (II) sind bekannte, zum Teil im Handel erhältliche Synthesechemikalien.

Die weiter als Ausgangsstoffe benötigten Arylamine der Formel (IV) sind ebenfalls bekannte, zum Teil im Handel erhältliche Synthesechemikalien (zur Synthese vgl. EP-A 325 247; J. Org. Chem. 44 (1979), 578-586 und die Herstellungsbeispiele).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. VorZugsweise kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kalium-carbonat, wie Natrium- und Kalium-tert-burylat, ferner aliphatische, aromatische oder heterocyclische Amine, beispielsweise Triethylamin, Trimethylamin, Dimethylanilin, Dimethylbenzylamin, Pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO) in Betracht.

Die Reaktionstemperaturen können bei dem erfindungsgemäßen Verfahren in einem größeren Bereich variiert werden. Im allgemeinen arbeitet man bei Temperaturen zwischen -30 °C und +80 °C, vorzugsweise bei Temperaturen zwischen -10 °C und +60 °C.

Das erfindungsgemäße Verfahren wird im allgemeinen unter Normaldruck durchgeführt. Es ist jedoch auch möglich, unter erhöhtem oder vermindertem Druck Zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden,

beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration Zur Totalunkrautbekämpffing z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere Zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethäbenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

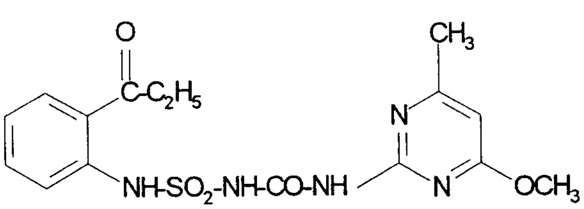

4,2 g (33 mMol) 2-Amino-4-methoxy-6-methyl-pyrimidin werden bei -5 °C unter Rühren zu einer Mischung aus 4,3 g (30 mMol) Chlorsulfonylisocyanat und 50 ml Methylenchlorid gegeben. Das Gemisch wird 30 Minuten bei 0 °C gerührt.

Dann werden 4,7 g (30 mMol) 2-Amino-propiophenon, 3,5 g (35 mMol) Triethylamin und 50 ml Methylenchlorid dazugegeben, und das Reaktionsgemisch wird 18 Stunden bei 20 °C nachgerührt.

Dann wird mit Wasser und mit 10%iger Salzsäure gewaschen, mit Natriumsulfat getrocknet und filtriert.

Das Filtrat wird im Vakuum eingeengt, der Rückstand wird durch Digerieren mit Ethanol zur Kristallisation gebracht und das Produkt durch Absaugen isoliert.

Man erhält 6,9 g (58,5% der Theorie) N-(4-Methoxy-6-methyl-pyrimidin-2-yl)-N'-(2-propionyl-phenylaminosulfonyl)-harnstoff vom Schmelzpunkt 126 °C.

Analog Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

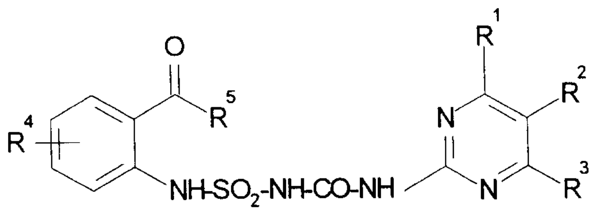

(I)

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 2 | $OCH_3$ | H | $OCH_3$ | H | $C_2H_5$ | 184 |
| 3 | $OCH_3$ | H | $OCH_3$ | H | $CH_3$ | 145 |
| 4 | $CH_3$ | H | $OCH_3$ | H | $CH_3$ | 172 |
| 5 | Cl | H | $OCH_3$ | H | $CH_3$ | 158 |
| 6 | $CH_3$ | H | $CH_3$ | H | $CH_3$ | 171 |
| 7 | H | H | $CH_3$ | H | $CH_3$ | 171 |
| 8 | $CH_3$ | H | $CH_3$ | H | $C_2H_5$ | 172 |
| 9 | Cl | H | $OCH_3$ | H | $C_2H_5$ | 152 |
| 10 | $OCH_3$ | H | $OCH_3$ | (6-)Cl | $C_2H_5$ | 162 |
| 11 | $OCH_3$ | H | $CH_3$ | (6-)Cl | $C_2H_5$ | 157 |
| 12 | $OCH_3$ | H | $OCH_3$ | (5-)Cl | $CH_3$ | 172 |
| 13 | $OCH_3$ | H | $OCH_3$ | (5-)Cl | $CH(CH_3)_2$ | 166 |
| 14 | $CH_3$ | H | $OCH_3$ | H | $CH(CH_3)_2$ | 149 |
| 15 | $OCH_3$ | H | $OCH_3$ | (6-)F | $CH_3$ | |
| 16 | $OCH_3$ | H | $OCH_3$ | (6-)$CH_3$ | $CH_3$ | 184 |
| 17 | $OCH_3$ | H | $OCH_3$ | (6-)Cl | $CH_3$ | 174 |
| 18 | $OCH_3$ | H | $OCH_3$ | (6-)$OCH_3$ | $CH_3$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 19 | $OCH_3$ | H | $OCH_3$ | (6-)$SCH_3$ | $CH_3$ | 162 |
| 20 | $OCH_3$ | H | $OCH_3$ | (6-)$COOCH_3$ | $CH_3$ | |
| 21 | $OCH_3$ | H | $OCH_3$ | (6-)F | $C_2H_5$ | 155 |
| 22 | $OCH_3$ | H | $OCH_3$ | (6-)$CH_3$ | $C_2H_5$ | 164 |
| 23 | $OCH_3$ | H | $OCH_3$ | (6-)$OCH_3$ | $C_2H_5$ | |
| 24 | $OCH_3$ | H | $OCH_3$ | (6-)$SCH_3$ | $C_2H_5$ | 220 |
| 25 | $OCH_3$ | H | $OCH_3$ | (6-)$COOCH_3$ | $C_2H_5$ | |
| 26 | $OCH_3$ | H | $OCH_3$ | (4-)Cl | n-$C_4H_9$ | 165 |
| 27 | $OCH_3$ | H | $OCH_3$ | (5-)Cl | $CH(CH_3)_2$ | 160 |
| 28 | $CH_3$ | H | $OCH_3$ | (6-)F | $CH_3$ | |
| 29 | $CH_3$ | H | $OCH_3$ | (6-)Cl | $CH_3$ | |
| 30 | $CH_3$ | H | $OCH_3$ | (6-)$CH_3$ | $CH_3$ | 136 |
| 31 | $CH_3$ | H | $OCH_3$ | (6-)$OCH_3$ | $CH_3$ | |
| 32 | $CH_3$ | H | $OCH_3$ | (6-)$SCH_3$ | $CH_3$ | |
| 33 | $CH_3$ | H | $OCH_3$ | (6-)$COOCH_3$ | $CH_3$ | |
| 34 | $CH_3$ | H | $OCH_3$ | (6-)F | $C_2H_5$ | 161 |
| 35 | $CH_3$ | H | $OCH_3$ | (6-)$CH_3$ | $C_2H_5$ | 212 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 36 | $CH_3$ | H | $OCH_3$ | (6-)$OCH_3$ | $C_2H_5$ | |
| 37 | $CH_3$ | H | $OCH_3$ | (6-)$SCH_3$ | $C_2H_5$ | |
| 38 | $CH_3$ | H | $OCH_3$ | (6-)$COOCH_3$ | $C_2H_5$ | |
| 39 | Cl | H | $OCH_3$ | (6-)F | $CH_3$ | |
| 40 | Cl | H | $OCH_3$ | (6-)Cl | $CH_3$ | 154 |
| 41 | Cl | H | $OCH_3$ | (6-)$CH_3$ | $CH_3$ | 145 |
| 42 | Cl | H | $OCH_3$ | (6-)$OCH_3$ | $CH_3$ | |
| 43 | Cl | H | $OCH_3$ | (6-)$SCH_3$ | $CH_3$ | |
| 44 | Cl | H | $OCH_3$ | (6-)$COOCH_3$ | $CH_3$ | |
| 45 | Cl | H | $OCH_3$ | (6-)F | $C_2H_5$ | 180 |
| 46 | Cl | H | $OCH_3$ | (6-)Cl | $C_2H_5$ | 184 |
| 47 | Cl | H | $OCH_3$ | (6-)$CH_3$ | $C_2H_5$ | 158 |
| 48 | Cl | H | $OCH_3$ | (6-)$OCH_3$ | $C_2H_5$ | |
| 49 | Cl | H | $OCH_3$ | (6-)$SCH_3$ | $C_2H_5$ | 196 |
| 50 | Cl | H | $OCH_3$ | (6-)$COOCH_3$ | $C_2H_5$ | |
| 51 | H | H | $CH_3$ | (6-)F | $CH_3$ | |
| 52 | H | H | $CH_3$ | (6-)Cl | $CH_3$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 53 | H | H | $CH_3$ | (6-)$CH_3$ | $CH_3$ | |
| 54 | H | H | $CH_3$ | (6-)$OCH_3$ | $CH_3$ | |
| 55 | H | H | $CH_3$ | (6-)$SCH_3$ | $CH_3$ | |
| 56 | H | H | $CH_3$ | (6-)$COOCH_3$ | $CH_3$ | |
| 57 | H | H | $CH_3$ | H | $C_2H_5$ | |
| 58 | H | H | $CH_3$ | (6-)F | $C_2H_5$ | |
| 59 | H | H | $CH_3$ | (6-)Cl | $C_2H_5$ | |
| 60 | H | H | $CH_3$ | (6-)$CH_3$ | $C_2H_5$ | |
| 61 | H | H | $CH_3$ | (6-)$OCH_3$ | $C_2H_5$ | |
| 62 | H | H | $CH_3$ | (6-)$SCH_3$ | $C_2H_5$ | |
| 63 | H | H | $CH_3$ | (6-)$COOCH_3$ | $C_2H_5$ | |
| 64 | H | H | $OCH_3$ | H | $CH_3$ | |
| 65 | H | H | $OCH_3$ | (6-)F | $CH_3$ | |
| 66 | H | H | $OCH_3$ | (6-)Cl | $CH_3$ | |
| 67 | H | H | $OCH_3$ | (6-)$CH_3$ | $CH_3$ | |
| 68 | H | H | $OCH_3$ | (6-)$SCH_3$ | $CH_3$ | |
| 69 | H | H | $OCH_3$ | (6-)$OCH_3$ | $CH_3$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 70 | H | H | $OCH_3$ | (6-)$COOCH_3$ | $CH_3$ | |
| 71 | H | H | $OCH_3$ | H | $C_2H_5$ | |
| 72 | H | H | $OCH_3$ | (6-)F | $C_2H_5$ | |
| 73 | H | H | $OCH_3$ | (6-)Cl | $C_2H_5$ | |
| 74 | H | H | $OCH_3$ | (6-)$OCH_3$ | $C_2H_5$ | |
| 75 | H | H | $OCH_3$ | (6-)$SCH_3$ | $C_2H_5$ | |
| 76 | H | H | $OCH_3$ | (6-)$CH_3$ | $C_2H_5$ | |
| 77 | H | H | $OCH_3$ | (6-)$COOCH_3$ | $C_2H_5$ | |
| 78 | $CH_3$ | H | $CH_3$ | (6-)F | $CH_3$ | |
| 79 | $CH_3$ | H | $CH_3$ | (6-)Cl | $CH_3$ | 158 |
| 80 | $CH_3$ | H | $CH_3$ | (6-)$CH_3$ | $CH_3$ | 128 |
| 81 | $CH_3$ | H | $CH_3$ | (6-)$SCH_3$ | $CH_3$ | |
| 82 | $CH_3$ | H | $CH_3$ | (6-)$OCH_3$ | $CH_3$ | |
| 83 | $CH_3$ | H | $CH_3$ | (6-)$COOCH_3$ | $CH_3$ | |
| 84 | $CH_3$ | H | $CH_3$ | (6-)F | $C_2H_5$ | 160 |
| 85 | $CH_3$ | H | $CH_3$ | (6-)Cl | $C_2H_5$ | |
| 86 | $CH_3$ | H | $CH_3$ | (6-)$OCH_3$ | $C_2H_5$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 87 | $CH_3$ | H | $CH_3$ | (6-)$SCH_3$ | $C_2H_5$ | 174 |
| 88 | $CH_3$ | H | $CH_3$ | (6-)$CH_3$ | $C_2H_5$ | 178 |
| 89 | $CH_3$ | H | $CH_3$ | (6-)$COOCH_3$ | $C_2H_5$ | |
| 90 | $CF_3$ | H | $CF_3$ | H | $CH_3$ | |
| 91 | $CF_3$ | H | $CF_3$ | (6-)F | $CH_3$ | |
| 92 | $CF_3$ | H | $CF_3$ | (6-)Cl | $CH_3$ | |
| 93 | $CF_3$ | H | $CF_3$ | (6-)$CH_3$ | $CH_3$ | |
| 94 | $CF_3$ | H | $CF_3$ | (6-)$SCH_3$ | $CH_3$ | |
| 95 | $CF_3$ | H | $CF_3$ | (6-)$OCH_3$ | $CH_3$ | |
| 96 | $CF_3$ | H | $CF_3$ | (6-)$COOCH_3$ | $CH_3$ | |
| 97 | $CF_3$ | H | $CF_3$ | H | $C_2H_5$ | |
| 98 | $CF_3$ | H | $CF_3$ | (6-)F | $C_2H_5$ | |
| 99 | $CF_3$ | H | $CF_3$ | (6-)Cl | $C_2H_5$ | |
| 100 | $CF_3$ | H | $CF_3$ | (6-)$OCH_3$ | $C_2H_5$ | |
| 101 | $CF_3$ | H | $CF_3$ | (6-)$SCH_3$ | $C_2H_5$ | |
| 102 | $CF_3$ | H | $CF_3$ | (6-)$CH_3$ | $C_2H_5$ | |
| 103 | $CF_3$ | H | $CF_3$ | (6-)$COOCH_3$ | $C_2H_5$ | |

Tabelle 1 (Fortsetzung)

| Bsp.-Nr. | $R^1$ | $R^2$ | $R^3$ | (Position-) $R^4$ | $R^5$ | Schmelz-punkt (°C) |
|---|---|---|---|---|---|---|
| 104 | $CF_3$ | H | $OCH_3$ | H | $CH_3$ | |
| 105 | $CF_3$ | H | $OCH_3$ | (6-)F | $CH_3$ | |
| 106 | $CF_3$ | H | $OCH_3$ | (6-)Cl | $CH_3$ | |
| 107 | $CF_3$ | H | $OCH_3$ | (6-)$CH_3$ | $CH_3$ | |
| 108 | $CF_3$ | H | $OCH_3$ | (6-)$SCH_3$ | $CH_3$ | |
| 109 | $CF_3$ | H | $OCH_3$ | (6-)$OCH_3$ | $CH_3$ | |
| 110 | $CF_3$ | H | $OCH_3$ | (6-)$COOCH_3$ | $CH_3$ | |
| 111 | $CF_3$ | H | $OCH_3$ | H | $C_2H_5$ | |
| 112 | $CF_3$ | H | $OCH_3$ | (6-)F | $C_2H_5$ | |
| 113 | $CF_3$ | H | $OCH_3$ | (6-)Cl | $C_2H_5$ | |
| 114 | $CF_3$ | H | $OCH_3$ | (6-)$OCH_3$ | $C_2H_5$ | |
| 115 | $CF_3$ | H | $OCH_3$ | (6-)$SCH_3$ | $C_2H_5$ | |
| 116 | $CF_3$ | H | $OCH_3$ | (6-)$CH_3$ | $C_2H_5$ | |
| 117 | $CF_3$ | H | $OCH_3$ | (6-)$COOCH_3$ | $C_2H_5$ | |
| 118 | $OCHF_2$ | H | $OCHF_2$ | H | $CH_3$ | |
| 119 | $OCHF_2$ | H | $OCHF_2$ | (6-)F | $CH_3$ | |
| 120 | $OCHF_2$ | H | $OCHF_2$ | (6-)Cl | $CH_3$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 121 | $OCHF_2$ | H | $OCHF_2$ | (6-)$CH_3$ | $CH_3$ | |
| 122 | $OCHF_2$ | H | $OCHF_2$ | (6-)$SCH_3$ | $CH_3$ | |
| 123 | $OCHF_2$ | H | $OCHF_2$ | (6-)$OCH_3$ | $CH_3$ | |
| 124 | $OCHF_2$ | H | $OCHF_2$ | (6-)$COOCH_3$ | $CH_3$ | |
| 125 | $OCHF_2$ | H | $OCHF_2$ | H | $C_2H_5$ | |
| 126 | $OCHF_2$ | H | $OCHF_2$ | (6-)F | $C_2H_5$ | |
| 127 | $OCHF_2$ | H | $OCHF_2$ | (6-)Cl | $C_2H_5$ | |
| 128 | $OCHF_2$ | H | $OCHF_2$ | (6-)$OCH_3$ | $C_2H_5$ | |
| 129 | $OCHF_2$ | H | $OCHF_2$ | (6-)$SCH_3$ | $C_2H_5$ | |
| 130 | $OCHF_2$ | H | $OCHF_2$ | (6-)$CH_3$ | $C_2H_5$ | |
| 131 | $OCHF_2$ | H | $OCHF_2$ | (6-)$COOCH_3$ | $C_2H_5$ | |
| 132 | $OCH_3$ | H | $NHCH_3$ | H | $CH_3$ | |
| 133 | $OCH_3$ | H | $NHCH_3$ | (6-)F | $CH_3$ | |
| 134 | $OCH_3$ | H | $NHCH_3$ | (6-)Cl | $CH_3$ | |
| 135 | $OCH_3$ | H | $NHCH_3$ | (6-)$CH_3$ | $CH_3$ | |
| 136 | $OCH_3$ | H | $NHCH_3$ | (6-)$SCH_3$ | $CH_3$ | |
| 137 | $OCH_3$ | H | $NHCH_3$ | (6-)$OCH_3$ | $CH_3$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 138 | $OCH_3$ | H | $NHCH_3$ | (6-)$COOCH_3$ | $CH_3$ | |
| 139 | $OCH_3$ | H | $NHCH_3$ | H | $C_2H_5$ | |
| 140 | $OCH_3$ | H | $NHCH_3$ | (6-)F | $C_2H_5$ | |
| 141 | $OCH_3$ | H | $NHCH_3$ | (6-)Cl | $C_2H_5$ | |
| 142 | $OCH_3$ | H | $NHCH_3$ | (6-)$OCH_3$ | $C_2H_5$ | |
| 143 | $OCH_3$ | H | $NHCH_3$ | (6-)$SCH_3$ | $C_2H_5$ | |
| 144 | $OCH_3$ | H | $NHCH_3$ | (6-)$CH_3$ | $C_2H_5$ | |
| 145 | $OCH_3$ | H | $NHCH_3$ | (6-)$COOCH_3$ | $C_2H_5$ | |
| 146 | $OC_2H_5$ | H | $NHCH_3$ | H | $CH_3$ | |
| 147 | $OC_2H_5$ | H | $NHCH_3$ | (6-)F | $CH_3$ | |
| 148 | $OC_2H_5$ | H | $NHCH_3$ | (6-)Cl | $CH_3$ | |
| 149 | $OC_2H_5$ | H | $NHCH_3$ | (6-)$CH_3$ | $CH_3$ | |
| 150 | $OC_2H_5$ | H | $NHCH_3$ | (6-)$SCH_3$ | $CH_3$ | |
| 151 | $OC_2H_5$ | H | $NHCH_3$ | (6-)$OCH_3$ | $CH_3$ | |
| 152 | $OC_2H_5$ | H | $NHCH_3$ | (6-)$COOCH_3$ | $CH_3$ | |
| 153 | $OC_2H_5$ | H | $NHCH_3$ | H | $C_2H_5$ | |
| 154 | $OC_2H_5$ | H | $NHCH_3$ | (6-)F | $C_2H_5$ | |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 155 | $OC_2H_5$ | H | $NHCH_3$ | (6-)Cl | $C_2H_5$ | |
| 156 | $OC_2H_5$ | H | $NHCH_3$ | (6-)$OCH_3$ | $C_2H_5$ | |
| 157 | $OC_2H_5$ | H | $NHCH_3$ | (6-)$SCH_3$ | $C_2H_5$ | |
| 158 | $OC_2H_5$ | H | $NHCH_3$ | (6-)$CH_3$ | $C_2H_5$ | |
| 159 | $OC_2H_5$ | H | $NHCH_3$ | (6-)$COOCH_3$ | $C_2H_5$ | |
| 160 | $OCH_3$ | H | $OCH_3$ | H | $CH_2SCH_3$ | 157 |
| 161 | $OCH_3$ | H | $OCH_3$ | H | $C_3H_7n$ | 156 |
| 162 | $CH_3$ | H | $OCH_3$ | H | $C_3H_7n$ | 149 |
| 163 | $CH_3$ | H | H | H | $C_3H_7n$ | 155 |
| 164 | $OCH_3$ | H | $OCH_3$ | (6-)F | $C_3H_7n$ | 142 |
| 165 | $OCH_3$ | H | $OCH_3$ | (6-)Cl | $C_3H_7n$ | 133 |
| 166 | $OCH_3$ | H | $OCH_3$ | (6-)$CH_3$ | $C_3H_7n$ | 72 |
| 167 | $OCH_3$ | H | $OCH_3$ | H | 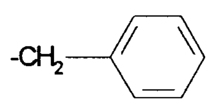 | 192 |
| 168 | $OCH_3$ | H | $OCH_3$ | H | 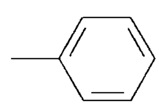 | 188 |
| 169 | $OCH_3$ | H | $CH_3$ | H | 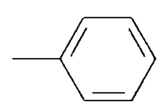 | 172 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 170 | $OCH_3$ | H | $OCH_3$ | H | $CH(CH_3)_2$ | 150 |
| 171 | $OCH_3$ | H | $OCH_3$ | H | $(CH_2)_3CH_3$ | 156 |
| 172 | $OCH_3$ | H | $OCH_3$ | H | 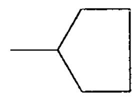 | 152 |
| 173 | $OCH_3$ | H | $OCH_3$ | (4-)Cl | 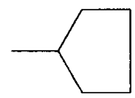 | 147 |
| 174 | $OCH_3$ | H | $OCH_3$ | (3-)F | $CH(CH_3)_2$ | |
| 175 | $OCH_3$ | H | $OCH_3$ | (5-)Cl | $CH_3$ | 163 |
| 176 | $OCH_3$ | H | $OCH_3$ | (4-)$CH_3$ | $CH(CH_3)_2$ | |
| 177 | $OCH_3$ | H | $OCH_3$ | (4-)$OCH_3$ | $CH(CH_3)_2$ | |
| 178 | $OCH_3$ | H | $OCH_3$ | (4-)$OCH_3$ | $CH_3$ | |
| 179 | $OCH_3$ | H | $CH_3$ | (4-)Cl | $(CH_2)_3CH_3$ | |
| 180 | $OCH_3$ | H | $CH_3$ | (4-)Cl | 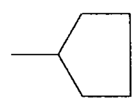 | |
| 181 | $OCH_3$ | H | $OCH_3$ | H | 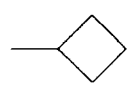 | 170 |
| 182 | $OCH_3$ | H | $OCH_3$ | H | 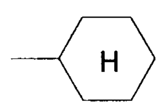 | 165 |
| 183 | $OCH_3$ | H | $OCH_3$ | H | t-$C_4H_9$ | 103 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 184 | $OCH_3$ | H | $OCH_3$ | H | 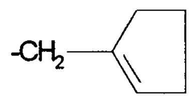 | 172 |
| 185 | $OCH_3$ | H | $OCH_3$ | H | 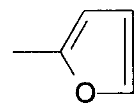 | 150 |
| 186 | $OCH_3$ | H | $OCH_3$ | (4-)$OCH_3$ | n-$C_4H_9$ | 149 |
| 187 | $OCH_3$ | H | $OCH_3$ | (5-)$OCH_3$ | i-$C_3H_7$ | 165 |
| 188 | $CH_3$ | H | $OCH_3$ | (4-)$OCH_3$ | $CH(CH_3)_2$ | 162 |
| 189 | $OCH_3$ | H | $OCH_3$ | (5-)$CH_3$ | $CH(CH_3)_2$ | 153 |
| 190 | $CH_3$ | H | $OCH_3$ | (5-)$CH_3$ | $CH(CH_3)_2$ | 164 |
| 191 | $OCH_3$ | H | $OCH_3$ | (6-)$C_2H_5$ | $CH(CH_3)_2$ | 152 |
| 192 | $OCH_3$ | H | $OCH_3$ | (6-)$CH(CH_3)_2$ | $CH(CH_3)_2$ | 176 |
| 193 | $OCH_3$ | H | $OCH_3$ | (6-)$C_4H_9$-s | $CH(CH_3)_2$ | 162 |
| 194 | $OCH_3$ | H | $OCH_3$ | (3-)$OCH_3$ | $CH_3$ | 147 |
| 195 | $OCH_3$ | H | $OCH_3$ | (5-)$OCH_3$ | n-$C_4H_9$ | 170 |
| 196 | $OCH_3$ | H | $OCH_3$ | (3-)$OCH_3$ | n-$C_4H_9$ | 158 |
| 197 | $OCH_3$ | H | $OCH_3$ | (5-)$C_2H_5$ | $CH(CH_3)_2$ | 176 |
| 198 | $OCH_3$ | H | $OCH_3$ | (4-) $NHCOOC_2H_5$ | $CH(CH_3)_2$ | 164 |

Tabelle 1 (Fortsetzung)

| Bsp.-Nr. | $R^1$ | $R^2$ | $R^3$ | (Position-) $R^4$ | $R^5$ | Schmelz-punkt (°C) |
|---|---|---|---|---|---|---|
| 199 | $OCH_3$ | H | $OCH_3$ | (6-)$C_2H_5$ | $CH_3$ | 159 |
| 200 | $OCH_3$ | H | $OCH_3$ | (6-)$C_2H_5$ | $C_2H_5$ | 160 |
| 201 | $OCH_3$ | H | $OCH_3$ | (6-)$C_2H_5$ | n-$C_4H_9$ | 116 |
| 202 | $OCH_3$ | H | $OCH_3$ | (6-)$CH(CH_3)_2$ | $C_2H_5$ | 161 |
| 203 | $OCH_3$ | H | $OCH_3$ | (6-)$CH(CH_3)_2$ | $CH_3$ | 157 |
| 204 | $OCH_3$ | H | $OCH_3$ | (6-) $NHCOOC_2H_5$ | $CH(CH_3)_2$ | 160 |
| 205 | Cl | H | $OCH_3$ | (6-)$C_2H_5$ | $C_2H_5$ | 148 |
| 206 | $OCH_3$ | H | $OCH_3$ | (6-)$CH_3$ | $-(CH_2)_3-Cl$ | 67 |
| 207 | $OCH_3$ | H | $OCH_3$ | H | 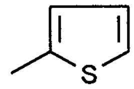 | 128 |
| 208 | $CH_3$ | H | $OCH_3$ | H | H | 105 |
| 209 | $OCH_3$ | H | $OCH_3$ | (6-)$CH_3$ | H | 147 |
| 210 | Cl | H | $OCH_3$ | H | H | 158 |
| 211 | Cl | H | $OCH_3$ | (6-)$CH_3$ | H | 194 |
| 212 | $OCH_3$ | H | $OCH_3$ | (6-)$C_3H_7$-n | $CH_3$ | 144 |
| 213 | $OCH_3$ | H | $OCH_3$ | (6-)$C_3H_7$-n | $C_2H_5$ | 151 |
| 214 | $OCH_3$ | H | $OCH_3$ | (6-)$C_3H_7$-n | $C_3H_7$-n | 148 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 215 | $CH_3$ | H | $OCH_3$ | (6-)$C_3H_7$-n | $CH_3$ | 133 |
| 216 | $CH_3$ | H | $OCH_3$ | (6-)$C_3H_7$-n | $C_2H_5$ | 144 |
| 217 | $CH_3$ | H | $OCH_3$ | (6-)$C_3H_7$-n | $C_3H_7$-n | 143 |
| 218 | $OCH_3$ | H | $OCH_3$ | (6-)$C_3H_7$-n | $CH(CH_3)_2$ | 140 |
| 219 | $CH_3$ | H | $OCH_3$ | (6-)$C_3H_7$-n | $CH(CH_3)_2$ | 135 |
| 220 | Cl | H | $OCH_3$ | H | $-CH_2SCH_3$ | 136 |
| 221 | $CH_3$ | H | $OCH_3$ | (6-)$CH_3$ | $C_3H_7$-n | 128 |
| 222 | Cl | H | $OCH_3$ | (6-)$CH_3$ | $C_3H_7$-n | 122 |
| 223 | $OCH_3$ | H | $OCH_3$ | (6-)$SC_2H_5$ | $C_3H_7$-n | 150 |
| 224 | Cl | H | $OCH_3$ | (6-)$SC_2H_5$ | $C_3H_7$-n | 125 |
| 225 | $CH_3$ | H | $CH_3$ | H | 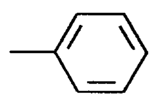 | 190 |
| 226 | Cl | H | $OCH_3$ | H | 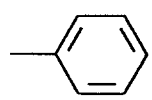 | 186 |
| 227 | $CH_3$ | Br | $CH_3$ | H | $C_2H_5$ | 178 |
| 228 | $CH_3$ | Br | $CH_3$ | (6-)F | $C_2H_5$ | 168 |
| 229 | $CH_3$ | Br | $CH_3$ | (6-)$CH_3$ | $C_2H_5$ | 154 |
| 230 | $OCH_3$ | H | $OCH_3$ | (6-)$SOC_2H_5$ | $C_3H_7$-n | 128 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 231 | $OCH_3$ | H | $OCH_3$ | (6-)$SO_2C_2H_5$ | $C_3H_7$-n | 125 |
| 232 | $OCH_3$ | H | $OCH_3$ | (6-)$SCH_3$ | $C_3H_7$-n | 148 |
| 233 | $CH_3$ | H | $OCH_3$ | (6-)$SCH_3$ | $C_3H_7$-n | 137 |
| 234 | $OCH_3$ | H | $OCH_3$ | (6-)$SO_2CH_3$ | $C_3H_7$-n | 132 |
| 235 | Cl | H | $OCH_3$ | H | $C_3H_7$-n | 129 |
| 236 | Cl | H | $OCH_3$ | (6-)$SCH_3$ | $C_3H_7$-n | 139 |
| 237 | Cl | H | $OCH_3$ | (6-)Cl | $C_3H_7$-n | 148 |
| 238 | $CH_3$ | H | $OCH_3$ | (6-)Cl | $C_3H_7$-n | 145 |
| 239 | $CH_3$ | H | $OCH_3$ | (6-)$SC_2H_5$ | $C_3H_7$-n | 168 |
| 240 | $CH_3$ | H | $OCH_3$ | (6-)F | $C_3H_7$-n | 149 |
| 241 | Cl | H | $OCH_3$ | (6-)F | $C_3H_7$-n | 148 |
| 242 | $OCH_3$ | H | $OCH_3$ | (6-)$SO_2CH_3$ | $CH_3$ | 182 |
| 243 | $CH_3$ | H | $OC_2H_5$ | H | $CH_3$ | 180 |
| 244 | $OCH_3$ | H | $OCH_3$ | (6-)Br | $C_2H_5$ | 185 |
| 245 | $CH_3$ | H | $OCH_3$ | (6-)Br | $C_2H_5$ | 169 |
| 246 | Cl | H | $OCH_3$ | (6-)Br | $C_2H_5$ | 161 |
| 247 | $CH_3$ | H | $CH_3$ | (6-)Br | $C_2H_5$ | 181 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 248 | $OCH_3$ | H | $OCH_3$ | (6-)Br | $CH_3$ | 195 |
| 249 | $CH_3$ | H | $OCH_3$ | (6-)Br | $CH_3$ | 191 |
| 250 | $OCH_3$ | H | $OCH_3$ | (6-)$CH_3$ | $-CH_2SCH_3$ | 148 |
| 251 | Cl | H | $OCH_3$ | (6-)$CH_3$ | $-CH_2SCH_3$ | 102 |
| 252 | $CH_3$ | H | $OCH_3$ | (6-)$CH_3$ | $-CH_2SCH_3$ | 85 |
| 253 | Cl | H | $OCH_3$ | (6-)$CH_3$ | $-CH_2SOCH_3$ | 112 |
| 254 | $OCH_3$ | H | $OCH_3$ | (6-)Br | $C_3H_7$-n | 174 |
| 255 | Cl | H | $OCH_3$ | (6-)Br | $C_3H_7$-n | 152 |
| 256 | $CH_3$ | H | $OCH_3$ | (6-)Br | $C_3H_7$-n | 148 |
| 257 | Cl | H | $OCH_3$ | (6-)Br | $CH_3$ | 182 |
| 258 | $CH_3$ | H | $CH_3$ | (6-)Br | $CH_3$ | 177 |
| 259 | Cl | H | $OC_2H_5$ | (6-)$SCH_3$ | $CH_3$ | 150 |
| 260 | Cl | H | $OC_2H_5$ | (6-)Cl | $C_2H_5$ | 174 |
| 261 | Cl | H | $OC_2H_5$ | (6-)Cl | $CH_3$ | 174 |
| 262 | Cl | H | $OC_2H_5$ | H | $C_2H_5$ | 124 |
| 263 | Cl | H | $CH_3$ | H | $C_2H_5$ | 161 |
| 264 | Cl | H | $CH_3$ | H | $CH_3$ | 196 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 265 | Cl | H | $CH_3$ | (6-)$CH_3$ | $CH_3$ | 201 |
| 266 | Cl | H | $CH_3$ | (6-)$SCH_3$ | $CH_3$ | 158 |
| 267 | Cl | H | $CH_3$ | (6-)$SCH_3$ | $C_2H_5$ | 140 |
| 268 | $OCH_3$ | H | $OCH_3$ | H | -$CH_2Cl$ | 244 |
| 269 | Cl | H | $OCH_3$ | H | -$CH_2Cl$ | 194 |
| 270 | $CH_3$ | H | $OCH_3$ | H | -$CH_2Cl$ | 177 |
| 271 | $CH_3$ | H | $CH_3$ | H | -$CH_2Cl$ | 156 |
| 272 | Cl | H | $CH_3$ | H | -$CH_2Cl$ | 140 |
| 273 | Cl | H | $OC_2H_5$ | H | -$CH_2Cl$ | 112 |
| 274 | $CH_3$ | H | $OC_2H_5$ | H | -$CH_2Cl$ | 175 |
| 275 | $OCH_3$ | H | $OCH_3$ | (6-)$CH_3$ | -$CH_2Cl$ | 178 |
| 276 | Cl | H | $OCH_3$ | (6-)$CH_3$ | -$CH_2Cl$ | 128 |
| 277 | $CH_3$ | H | $OCH_3$ | (6-)$CH_3$ | -$CH_2Cl$ | 153 |
| 278 | $CH_3$ | H | $CH_3$ | (6-)$CH_3$ | -$CH_2Cl$ | 164 |
| 279 | Cl | H | $CH_3$ | (6-)$CH_3$ | -$CH_2Cl$ | 171 |
| 280 | Cl | H | $OC_2H_5$ | (6-)$CH_3$ | -$CH_2Cl$ | 136 |
| 281 | $CH_3$ | H | $OC_2H_5$ | (6-)$CH_3$ | -$CH_2Cl$ | 155 |

Tabelle 1 (Fortsetzung)

| Bsp.-Nr. | $R^1$ | $R^2$ | $R^3$ | (Position-) $R^4$ | $R^5$ | Schmelzpunkt (°C) |
|---|---|---|---|---|---|---|
| 282 | $OCH_3$ | H | $OCH_3$ | (6-)Cl | $-CH_2Cl$ | 155 |
| 283 | Cl | H | $OCH_3$ | (6-)F | $-CH_2Cl$ | 158 |
| 284 | $OCH_3$ | H | $OCH_3$ | (6-)Br | $-CH_2Cl$ | 155 |
| 285 | Cl | H | $OCH_3$ | (6-)Br | $-CH_2Cl$ | 105 |

Ausgangsstoffe der Formel (IV):

Beispiel (IV-1)

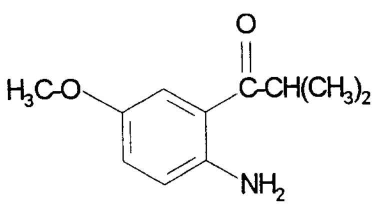

Eine Mischung aus 100 ml (0,1 Mol) Bortrichlorid und 100 ml 1,2-Dichlorethan wird auf 0 °C abgekühlt. Bei dieser Temperatur werden 12,3 g (0,1 Mol) 4-Methoxyanilin innerhalb von 15 Minuten unter Rühren zugetropft. Dann wird eine Lösung von 10,4 g (0,15 Mol) Isobutyronitril in 100 ml 1,2-Dichlorethan zugetropft und nach 5 Minuten Rühren werden bei 0 °C 14,7 g (0,11 Mol) Aluminiumtrichlorid portionsweise zur Mischung gegeben. Das Reaktionsgemisch wird dann 5 Stunden bei 80 °C gerührt, anschließend mit Eis gekühlt und tropfenweise mit 300 ml Wasser versetzt. Die organische Phase wird dann abgetrennt und die wäßrige Phase noch dreimal mit Methylenchlorid nachextrahiert. Die vereinigten organischen Phasen werden über Natriumsulfat getrocknet, filtriert und das Filtrat eingeengt. Der Rückstand wird säulenchromatographisch aufgetrennt (Kieselgel; Hexan/Essigsäureethylester- Vol.: 4:1).

Man erhält 5,3 g (27,5% der Theorie) 4-Methoxy-2-isobutyroyl-anilin.

Beispiel (IV-2)

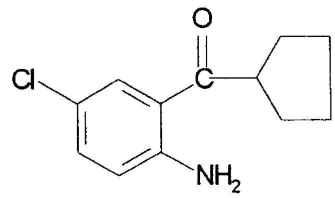

10,9 g (72 mMol) 2-Cyano-4-chlor-anilin werden in 30 ml Diethylether gelöst und auf 0 °C abgekühlt. Bei dieser Temperatur werden 100 ml einer zweimolaren Lösung von Cyclopentylmagnesiumchlorid in Diethylether zugetropft und die Reaktionsmischung wird dann noch 4,5 Stunden bei 20 °C gerührt. Nach Abkühlen auf 0 °C werden 150 ml 10%ige Salzsäure zugetropft und die Mischung wird eine Stunde bei 20 °C gerührt. Nach erneutem Abkühlen auf 0 °C werden 25 g Natriumhydroxid zur Mischung gegeben, die organische Phase abgetrennt, die wäßrige Phase dreimal mit Diethylether nachextrahiert, die vereinigten organischen Phasen über Natriumsulfat getrocknet und eingeengt.

Der Rückstand wird säulenchromatographisch aufgetrennt (Kieselgel; Hexan/Essigsäureethylester- Vol.: 4:1).

Man erhält 11,9 g (74% der Theorie) 4-Chlor-2-cyclopentanoyl-anilin vom Schmelzpunkt 73 °C.

Analog Beispiel (IV-1) oder (IV-2) können beispielsweise auch die in der nachstehenden Tabelle 2 aufgeführten Verbindungen der Formel (IV) hergestellt werden.

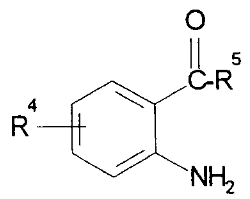

(IV)

Tabelle 2: Beispiele für die Verbindungen der Formel (IV)

| Bsp.-Nr. | (Position-) $R^4$ | $R^5$ | Schmelzpunkt (°C) |
|---|---|---|---|
| IV-3 | H | $CH(CH_3)_2$ | (Oel) |
| IV-4 | (4-)Cl | $CH(CH_3)_2$ | 64°C |
| IV-5 | H | $(CH_2)_3CH_3$ | (Oel) |
| IV-6 | H | 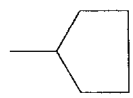 | 59°C |
| IV-7 | (4-)Cl | $CH_3$ | |
| IV-8 | (4-)Cl | $(CH_2)_3CH_3$ | 69°C |
| IV-9 | (4-)Cl | 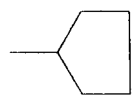 | 73°C |
| IV-10 | (5-)Cl | $CH(CH_3)_2$ | |
| IV-11 | (3-)F | $CH(CH_3)_2$ | (Oel) |
| IV-12 | (5-)Cl | $CH_3$ | |
| IV-13 | (4-)$CH_3$ | $CH(CH_3)_2$ | |
| IV-14 | (4-)$OCH_3$ | $CH(CH_3)_2$ | (Oel) |
| IV-15 | (4-)$OCH_3$ | $CH_3$ | (Oel) |
| IV-16 | H | $CH_2SCH_3$ | (Oel) |
| IV-17 | H | $C_3H_7n$ | 46°C |
| IV-18 | (6-)F | $C_3H_7n$ | |

Tabelle 2 (Fortsetzung)

| Bsp.-Nr. | (Position-) $R^4$ | $R^5$ | Schmelzpunkt (°C) |
|---|---|---|---|
| IV-19 | (6-)Cl | $C_3H_7n$ | |
| IV-20 | (6-)$CH_3$ | $C_3H_7n$ | |
| IV-21 | H | 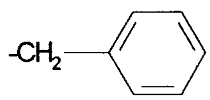 | 86°C |
| IV-22 | (6-)Cl | $C_2H_5$ | |
| IV-23 | (6-)F | $C_2H_5$ | |
| IV-24 | H | 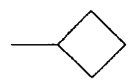 | $^1$H-NMR: d = 4,03 ppm |
| IV-25 | H | 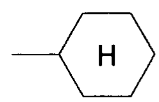 | 68°C-72°C |
| IV-26 | H | t-$C_4H_9$ | $^1$H-NMR: d = 1,29 ppm |
| IV-27 | (6-)Cl | $CH_3$ | |
| IV-28 | (6-)$CH_3$ | $CH_3$ | 48 |
| IV-29 | (6-)$SCH_3$ | $C_3H_7$-n | |
| IV-30 | (6-)$SCH_3$ | $CH_3$ | |
| IV-31 | (6-)$CH_3$ | -$CH_2SCH_3$ | |
| IV-32 | (6-)Br | $C_3H_7$-n | 59 |

Anwendungsbeispiele

In den Anwendungsbeispielen wird die folgende Verbindung (A) als Vergleichssubstanz herangezogen:

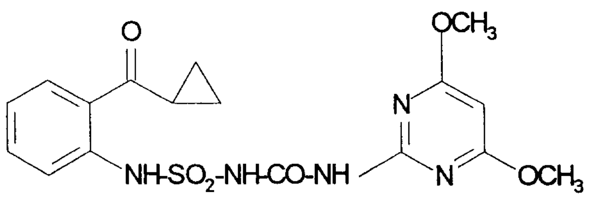

(A)

N-(4,6-Dimethoxy-pyrimidin-2-yl)-N'-(2-cyclopropyl-carbony-phenylaminosulfonyl)-harnstoff (bekannt aus EP-A 463287/US-P 5009699).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = unbehandelte Kontrolle/keine Wirkung
100 % = totale Vernichtung.

In diesem Test zeigen beispielsweise die Verbindungen gemäß Herstellungsbeispiel 1 und 2 bei sehr guter Verträglichkeit gegenüber Kulturpflanzen, wie z.B. Weizen, erheblich stärkere Wirkung gegen Unkräuter als die bekannte Verbindung (A).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die Verbindungen gemäß Herstellungsbeispiel 1 und 2 bei sehr guter Verträglichkeit gegenüber Kulturpflanzen, wie z.B. Weizen, erheblich stärkere Wirkung gegen Unkräuter als die bekannte Verbindung (A).

## Patentansprüche

1. Substituierte Phenylaminosulfonylharnstoffe der allgemeinen Formel (I)

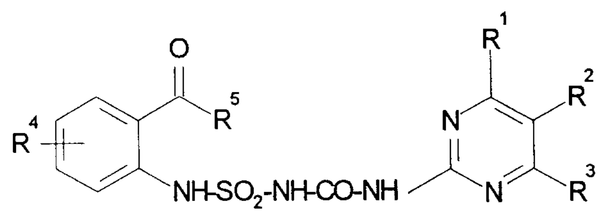

(I)

in welcher

$R^1$ für Wasserstoff, Halogen oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylamino oder Dialkylamino mit jeweils 1 bis 4 Kohlenstoffatomen in den einzelnen Alkylgruppen steht,

$R^2$ für Wasserstoff oder Halogen steht,

$R^3$ für Halogen oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylamino oder Dialkylamino mit jeweils 1 bis 4 Kohlenstoffatomen in den einzelnen Alkylgruppen steht,

$R^4$ für Wasserstoff, Amino, Halogen, Formyl oder für jeweils gegebenenfalls durch Halogen oder $C_1$-$C_3$-Alkoxy substituiertes Alkyl, Alkoxy, Alkylthio, Alkylsulfinyl, Alkylsulfonyl, Alkylcarbonyl, Alkoxycarbonyl, Alkylcarbonylamino, Alkoxycarbonylamino oder Alkylsulfonylamino mit jeweils 1 bis 4 Kohlenstoffatomen in den einzelnen Alkylgruppen steht, und

$R^5$ für Wasserstoff oder für gegebenenfalls durch Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 8 Kohlenstoffatomen, für jeweils gegebenenfalls durch Halogen und/oder $C_1$-$C_4$-Alkyl substituiertes Cycloalkyl, Cycloalkyl-$C_1$-$C_4$-alkyl, Cycloalkenyl oder Cycloalkeny-$C_1$-$C_4$-alkyl mit jeweils 4 bis 7 Kohlenstoffatomen in den Cycloalkyl- oder Cycloalkenylgruppen, für jeweils gegebenenfalls durch Halogen, $C_1$-$C_4$-Alkyl oder $C_1$-$C_4$-Halogenalkyl substituiertes Phenyl oder Phenyl-$C_1$-$C_4$-alkyl oder für jeweils gegebenenfalls durch Halogen, $C_1$-$C_4$-Alkyl oder $C_1$-$C_4$-Halogen-alkyl substituiertes Heterocyclyl oder Heterocyclyl-$C_1$-$C_4$-alkyl mit 2 bis 6 Kohlenstoffatomen und 1 bis 3 Stickstoffatomen und/oder einem Sauerstoff- oder Schwefelatom steht,

sowie Salze der Verbindungen der Formel (I).

2. Verbindungen der Formel (I) gemäß Anspruch 1, dadurch gekennzeichnet, daß darin

$R^1$ für Wasserstoff, Fluor, Chlor, Brom oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylamino oder Dialkylamino mit jeweils 1 bis 3 Kohlenstoffatomen in den einzelnen Alkylgruppen steht,

$R^2$ für Wasserstoff, Fluor, Chlor oder Brom steht,

$R^3$ für Fluor, Chlor, Brom oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylamino oder Dialkylamino mit jeweils 1 bis 3 Kohlenstoffatomen in den einzelnen Alkylgruppen steht,

$R^4$ für Wasserstoff Amino` Fluor, Chlor, Brom, Formyl oder für jeweils gegebenenfalls durch Fluor, Chlor, Methoxy oder Ethoxy substituiertes Alkyl, Alkoxy, Alkylthio, Alkylsulfinyl, Alkylsulfonyl, Alkylcarbonyl, Alkoxycarbonyl, Alkylcarbonylamino, Alkoxycarbonylamino oder Alkylsulfonylamino mit jeweils 1 bis 3 Kohlenstoffatomen in den einzelnen Alkylgruppen steht, und

$R^5$ für Wasserstoff oder für gegebenenfalls durch Fluor, Chlor, Brom, $C_1$-$C_3$-Alkoxy, $C_1$-$C_3$-Alkylthio, $C_1$-$C_3$-Alkylsulfinyl oder $C_1$-$C_3$-Alkylsulfonyl substituiertes Alkyl mit 1 bis 6 Kohlenstoffatomen, für gegebenenfalls durch Fluor, Chlor, Brom und/oder $C_1$-$C_3$-Alkyl substituiertes Cycloalkyl, Cycloalkyl-$C_1$-$C_3$-alkyl, Cycloalkenyl oder Cycloalkenyl-$C_1$-$C_3$-alkyl mit jeweils 4 bis 6 Kohlenstoffatomen in den Cycloalkyl- oder Cycloalkenylgruppen, für jeweils gegebenenfalls durch Fluor, Chlor, Brom, $C_1$-$C_3$-Alkyl oder $C_1$-$C_3$-Halogenalkyl substituiertes Phenyl oder Phenyl-$C_1$-$C_3$-alkyl oder für jeweils gegebenenfalls durch Fluor, Chlor, Brom, $C_1$-$C_3$-Alkyl oder $C_1$-$C_3$-Halogenalkyl substituiertes Heterocyclyl oder Heterocyclyl-$C_1$-$C_3$-alkyl mit 3

bis 5 Kohlenstoffatomen und 1 bis 3 Stickstoffatomen und/oder einem Sauerstoff- oder Schwefelatom steht,

sowie deren Salze.

**3.** Verbindungen der Formel (I) gemäß Anspruch 1, dadurch gekennzeichnet, daß darin

$R^1$ für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht,

$R^2$ für Wasserstoff steht,

$R^3$ für Chlor, Methyl, Ethyl, Trifluormethyl, Methoxy, Ethoxy, Difluormethoxy, Methylthio, Ethylthio, Methylamino, Ethylamino oder Dimethylamino steht,

$R^4$ für Wasserstoff, Amino, Fluor, Chlor, Brom, Methyl, Trifluormethyl, Methoxy, Methylthio, Methylsulfinyl, Methylsulfonyl, Acetyl oder Methoxycarbonyl steht, und

$R^5$ für Wasserstoff oder für jeweils gegebenenfalls durch Fluor und/oder Chlor substituiertes Methyl, Ethyl, n- oder i-Propyl, n-, i- oder s-Butyl, n-, i- oder s-Pentyl, für Methoxymethyl, Methoxyethyl, Ethoxymethyl, Ethoxyethyl, Methylthiomethyl, Methylthioethyl, Ethylthiomethyl, Ethylthioethyl, Methylsulfinylmethyl, Ethylsulfinylmethyl, Methylsulfonylmethyl, Ethylsulfonylmethyl, oder für jeweils gegebenenfalls durch Fluor, Chlor und/oder Methyl substituiertes Cyclobutyl, Cyclobutylmethyl, Cyclopentyl, Cyclopentylmethyl, Cyclopentenylmethyl, Cyclohexyl, Cyclohexenylmethyl, Phenyl, Benzyl, Phenylethyl, Furyl, Furylmethyl, Thienyl, Thienylmethyl, Tetrahydrofuryl oder Tetrahydrofurylmethyl steht,

und deren Salze.

**4.** Verbindungen der Formel (Ia)

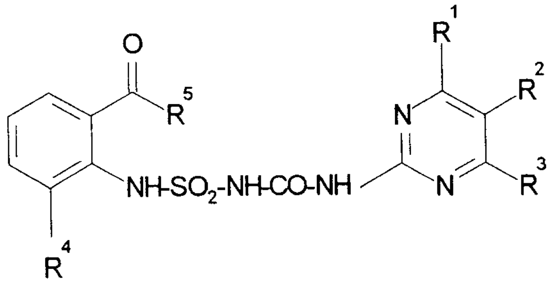

(Ia)

in welcher

$R^1$, $R^2$, $R^3$, $R^4$ und $R^5$ die in Anspruch 3 angegebenen Bedeutungen haben,

sowie deren Salze.

**5.** Verfahren zur Herstellung von substituierten Phenylaminosulfonylharnstoffen der Formel (I) und deren Salzen gemäß Anspruch 1, dadurch gekennzeichnet, daß man Aminopyrimidine der Formel (II)

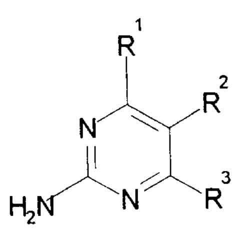

(II)

in welcher

$R^1$, $R^2$ und $R^3$ die in Anspruch 1 angegebene Bedeutung haben,

mit Chlorsulfonylisocyanat, gegebenenfalls in Gegenwart eines Verdünnungsmittels, umsetzt und die hierbei gebildeten Chlorsulfonylharnstoffe der allgemeinen

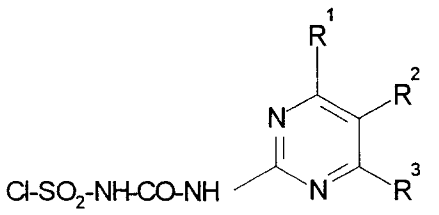

(III)

Formel (III), in welcher
$R^1$, $R^2$ und $R^3$ die in Anspruch 1 angegebene Bedeutung haben,
mit Arylaminen der allgemeinen Formel (IV),

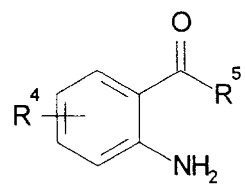

(IV)

in welcher
$R^4$ und $R^5$ die in Anspruch 1 angegebene Bedeutung haben,
gegebenenfalls in Gegenwart eines Säureakzeptors und gegebenenfalls in Gegenwart eines Verdünnungsmittels umsetzt und gegebenenfalls die so erhaltenen Verbindungen der Formel (I) nach üblichen Methoden in Salze überführt.

**6.** Herbizide Mittel, gekennzeichnet durch einen Gehalt an mindestens einer Verbindung der Formel (I) gemäß Anspruch 1.

**7.** Verwendung von Verbindungen der allgemeinen Formel (I) gemäß Anspruch 1 zur Bekämpfung von unerwünschtem Pflanzenwachstum.

**8.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**9.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung
EP 94 11 6723

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | CHEMICAL ABSTRACTS, vol. 120, no. 11, 14. März 1994, Columbus, Ohio, US; abstract no. 127786n, KAWAI,K. 'Compounds for prevention of phytotoxicity of sulfamoylurea herbicides on rice plant' Seite 388 ; * Zusammenfassung * | 1-9 | C07D239/545<br>A01N47/36 |
| X | & JP-A-05 255 023 (AMERICAN CYANAMID COMPANY) 5. Oktober 1993 --- | 1-9 | |
| D,X | EP-A-0 463 287 (AMERICAN CYANAMID COMPANY) 2. Januar 1992 --- | 1-9 | |
| D,X | EP-A-0 264 467 (PPG INDUSTRIES, INC.) 27. April 1988 --- | 1-9 | |
| Y | EP-A-0 303 114 (HOECHST AG) 15. Februar 1989 --- | 1-9 | |
| X | DE-A-33 00 569 (CIBA-GEIGY AG) 21. Juli 1983 * siehe insbesondere Seite 15, Beispiel 2 * --- | 1-9 | **RECHERCHIERTE SACHGEBIETE (Int.Cl.6)**<br>C07D<br>A01N |
| Y | DE-A-32 43 533 (SANDOZ AG) 9. Juni 1983 --- | 1-9 | |
| Y | EP-A-0 342 569 (HOECHST AG) 23. November 1989 --- | 1-9 | |
| Y | EP-A-0 004 163 (E.I. DU PONT DE NEMOURS AND COMPANY) 19. September 1979 --- | 1-9 | |

-/--

Der vorliegende Recherchenbericht wurde für alle Patentansprüche erstellt

| Recherchenort | Abschlußdatum der Recherche | Prüfer |
|---|---|---|
| MÜNCHEN | 23. Februar 1995 | Stellmach, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 94 11 6723

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | DRABER,W.,FUJITA,T. 'Rational Approaches to Stucture, Activity and Ecotoxicology of Agrochemicals'<br>1992 , CRC PRESS , BOCA RATON,FLORIDA<br>* siehe Chapter 15, T.A.ANDREA et al., Seite 373-395, insbesondere Seite 385, Tabelle 8 *<br>----- | 1-9 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| MÜNCHEN | 23. Februar 1995 | Stellmach, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)